# EUROPEAN PATENT APPLICATION

(11) **EP 3 888 466 A1**
(43) Date of publication of application: **06.10.2021**
(21) Application number: 19896977.6
(22) Date of filing: 10.12.2019
(51) Int. Cl.: A21D 2/08, A21D 13/06

(54) **PREPARATION OF INGREDIENTS FOR PRODUCING BREAD WITH REDUCED GLYCEMIC INDEX**

(30) Priority: 11.12.2018 ES 201831203
(71) Applicant: Universidad de Granada, 18071 Granada (ES); Panes Funcionales Antonio Soto, S.L., 18100 Armilla (ES)
(72) Inventor: GARCÍA-VILLANOVA RUIZ, Belén, 18071 Granada (ES); GUERRA HERNÁNDEZ, Eduardo, 18071 Granada (ES); SOTO FERNÁNDEZ, Victoriano, 18100 Armilla (ES); SOTO FERNÁNDEZ, Antonio, 18100 Armilla (ES)
(74) Representative: Sahuquillo Huerta, Jesús
(86) International application number: PCT/ES2019/070836
(87) International publication number: WO 2020/120815

(57) **Abstract**

The invention relates to a preparation of ingredients comprising alpha-cyclodextrin and other ingredients which permit the production of a mass suitable for processing in the traditional manner and the production of a bread with reduced glycemic index.

## Description

### TECHNICAL FIELD

The present invention is comprised in the sector of bakery products, both in the field of dough treatment and baking processes by means of the addition of ingredients, and in the field of products with a modified nutritional value.

### STATE OF THE ART

### Drawbacks associated with the glycemic index in food

The role of the glycemic index in different diseases such as diabetes and insulin resistance, age-related macular degeneration and cataracts, overweight and obesity, metabolic syndrome, colon, rectal, and breast cancers, cardiovascular disease, and total mortality is currently being investigated. The results found to date are not conclusive, but part of the epidemiological studies show an improvement in disease when diets with a low glycemic index are consumed.

### Relationship between alpha-cvclodextrins and the glycemic index

In the Scientific Opinion of the Panel on Dietetic Products, Nutrition and Allergies of the European Food Safety Authority (EFSA), according to two intervention studies, alpha-cyclodextrin added to starch showed a significant effect on the postprandial glycemic response without disproportionately increasing the postprandial insulin response. This did not occur when it was added to sucrose. On the basis of the data presented, the Panel concluded that a cause and effect relationship is established between the consumption of alpha-cyclodextrin with starch-containing meals and reduction of postprandial glycemic response. The panel considers that to obtain the claimed effect, at least 5 g of alpha-cyclodextrin per 50 g of starch should be consumed. The target population of this health claim is adults who wish to reduce their postprandial glycemic response [EFSA. Scientific Opinion on the substantiation of health claims related to alpha-cyclodextrin and reduction of post-prandial glycaemic responses (ID 2926, further assessment) pursuant to Article 13(1) of Regulation (EC) No 1924/2006. EFSA Journal 2012 10(6):2713].

### Glycemic index of bread

Breads belong to a group of foods that increase the insulin response; the carbohydrates of these products are represented by gelatinised starch and dextrins, which are readily digestible by human amylases and, accordingly, induce high glycemic responses. Insoluble fibre does not modify the gastric emptying rate, and therefore, the glycemic response induced by wholemeal breads is similar to that of white breads. If whole wheat grains are added to wholemeal breads, the glycemic index is less than that of bread produced with wholemeal flours, but other factors, such as the different particle size in flours (grain size measurement) used or the amylose percentage in starch, also have an effect. The incorporation of a large amount of soluble fibre increases the viscosity of the alimentary bolus, limits access to amylolytic enzymes, and reduces the diffusion of glucose through the mucosa, which causes these products to present a lower glycemic index. Rye breads produced with sourdough and the presence of organic acids seem to lower postprandial blood glucose and blood insulin.

The modification of the size and physical shape of bread has been used for producing breads with a smaller volume and higher density of nutrients creating a physical barrier impeding amylase accessibility.

Substituting wheat flour with flour from other cereals (corn, rice, rye, or even pseudocereals, such as buckwheat, amaranth, and quinoa) also produces changes in the food's glycemic index, although the magnitude of said changes will depend on the amount of flour added. The addition of sourdough starter also lowers the glycemic index due to the mentioned presence of organic acids and due to the increase in the amount of resistant starch.

*Diabetes mellitus* is a complex metabolic endocrine disorder in which there is a predominant disruption in the metabolism of carbohydrates due to a pancreatic insulin reduction, a reduction in the sensitivity of peripheral insulin receptors, or both factors. It furthermore presents with disruptions in lipid and protein metabolism and with the development of long-term vascular complications.

Diabetics present a baseline, i.e., fasting blood glucose of 126 mg/dl, and pre-diabetics, which is a group of patients who, without meeting the criteria for diabetes mellitus, have a higher risk of being diabetic, present fasting glucose levels of 100-126 mg/dl.

### Problems for the pre-diabetic and diabetic population

*Diabetes mellitus* is one of the main causes of mortality in developed and developing countries. In Spain, diabetes affects 2,600,000 people between the ages of 30 and 89. The WHO estimates that over 180 million people worldwide suffer diabetes and it is calculated that these values will double by 2030. The prevalence of diabetes mellitus in the Spanish elderly over 65 years of age is 16-23% of the population.

### Methods of producing bread with reduced glycemic index

The applicants consider that the document closest to the present invention is patent application number AU 2004200580A1, which describes a method for reducing the glycemic index of a pastry product with sugar, milk, and fat.

However, with the information provided in the document, the production of bread with the standard ingredients, such as wheat flour, water, salt, yeast, and coadjuvants, and produced by means of conventional processing, including the phases of kneading, fermentation, and baking, is not viable since the incorporation of alpha-cyclodextrins in the production process causes the following problems:
- Bread with a smaller volume
- Bread without ears
- Bread with little crust and no uniformity
- Bread devoid of air hole formation
- Bread without elasticity
- Bread with an irregular colour and very reddish, even in the crumb
- Bread very rubbery when chewed and requiring excessive swallowing effort

In view of the state of the art known by the applicants, there is still no known method for producing bread with conventional processing and comprising an amount of alpha-cyclodextrin sufficient for reducing the glycemic index, according to the specifications of EU regulation 536/2013 (according to the current text of 11 December 2018) and with the characteristics typical of the product.

After thorough research, the inventors of the present invention have surprisingly discovered that the addition of gluten up to a certain amount of total gluten allows producing or obtaining bread with conventional processing and comprising an amount of alpha-cyclodextrin sufficient for reducing the glycemic index, according to the specifications of the EU regulation 536/2013 (according to the current text of 11 December 2018) and having the characteristics typical of the bread obtained by means of conventional processing, i.e.: volume, crust and uniformity thereof, ear, air hole formation, colour, texture and organoleptic characteristics, including flavour, similar or identical to those of bread produced according to conventional processing but to which there has not been added or which does not comprise alpha-cyclodextrin.

### BRIEF DESCRIPTION OF THE INVENTION

The objective of the present invention is to produce bread with conventional processing with reduced glycemic index.

In a first aspect, the present invention relates to a preparation of ingredients comprising alpha-cyclodextrin and other ingredients which allow dough suitable for processing in a conventional manner to be obtained and bread with reduced glycemic index to be produced. The ingredients and the amounts comprised in the preparation considerably affect the structure and sensory characteristics of the bread produced and make it unnecessary to incorporate additives to enable maintaining the process of producing bread in a conventional manner.

In a second aspect, the present invention relates to dough obtained from the preparation of ingredients of the first aspect of the invention, i.e., dough obtained from a preparation of ingredients of the present invention.

In a third aspect, the present invention relates to bread obtained from a preparation of ingredients or dough of the present invention.

The present invention also relates to bread which comprises at least 5 g of alpha-cyclodextrin per 50 g of starch and can be produced by baking the dough obtained from the preparation of ingredients of the first aspect of the invention. This bread allows food for the pre-diabetic and diabetic population and for the general population to be improved.

In a fourth aspect, the present invention also describes prebaked bread, obtained from the dough of the second aspect of the invention, i.e., prebaked bread obtained from dough of the present invention. In this fourth aspect, the present invention also relates to prebaked bread obtained from a preparation of ingredients of the present invention.

In a final aspect, the present invention relates to the use of gluten (more preferably added gluten) in combination with alpha-cyclodextrin for the preparation of bread with reduced glycemic index.

### DETAILED DESCRIPTION OF THE INVENTION

### Definitions

Throughout the present invention, the term "bread", without any other descriptor, denotes the product resulting from baking dough comprising a mixture of wheat flour and drinking water, with or without the addition of edible salt, fermented by microorganism species characteristic of bread fermentation (Royal Decree (hereinafter RD) 285/1999 of 22 February. Technical Health Regulation for the production, circulation, and sale of bread and special breads, according to the current text of 11 December 2018).

"Ordinary bread" shall be understood to mean bread produced with wheat flour and to which there may be added the technological coadjuvants and additives authorised according to RD 285/1999. Technical Health Regulation for the production, circulation, and sale of bread and special breads, according to the current text of 11 December 2018.

The concept of "conventionally processed bread" refers to any type of bread, including among its ingredients at least wheat flour, drinking water, salt, and natural starters, to which the ingredients necessary for its technological and organoleptic development have been added, and which has been produced with at least the kneading, fermentation, and baking processes, including breads produced in any type of intermediate prebaking process.

"Special bread" is understood to mean that bread which meets any of the conditions indicated under Art. 4 of RD 285/1999, according to the current text of 11 December 2018.

In particular, "special bread" shall be understood to mean bread the production of which meets the following conditions:
- Processed flour is used as a raw material.
- Bran and wheat gluten have been added.

As it is used herein, "Pullman bread" and the plural form thereof take on the meaning they commonly have in the state of the art, i.e., it is bread that is fermented and baked in a mould which imparts to the bread the format or shape that said mould has.

The name "processed flour" comprises any flour to which any product that raises its nutritional value has been added. In other words, this name comprises any flour the composition of which includes other ingredients or which has been obtained by following processes which go beyond those typical of the process of milling, whether for the flour or for the raw materials used. In particular, this term comprises those flours enriched with the products and in the maximum proportions set in the supplementary positive lists in Chapter XXXVI of RD 677/2016 of 16 December, according to the current text of 11 December 2018.

In the present invention the gluten naturally present in flour will be distinguished from the gluten that is added, or "added gluten", as an ingredient in bread for achieving the technical characteristics necessary for the incorporation of alpha-cyclodextrin. In this context, the term "total gluten" will be used to refer to the sum of gluten naturally present in flour and added gluten.

In this context, "bread improver" denotes substances which facilitate or improve the breadmaking process, in which a series of technological coadjuvants used for a specific technological purpose and which can give rise to the involuntary presence in the final product of residues of the substance itself or of its byproducts are included, provided that they do not present any health risk.

"Sourdough starter" is a product obtained from the fermentation of the flour in the presence of drinking water by microorganisms from the environment and from the flour, or bread starters prepared for their incorporation in the dough directly. Preferably, the sourdough starter is a starter made up of flour and drinking water which does not contain any type of added yeast. In the flour itself, there are a number of yeasts and bacteria which cause the fermentation of sourdough starters in a spontaneous or natural manner.

"Kneading" is any process consisting of repeatedly moving and pressing one or more solid substances with a liquid, preferably water, until a homogeneous, compact, and soft dough is formed, allowing the absorption of water, the incorporation of air, and the formation of gluten. In this context, "moulding" is the process of forming balls (pieces of dough with a considerably spherical shape) with the dough to obtain homogeneous dough.

The term "fermentation" denotes any process of converting sugars into carbon dioxide and water carried out by the microorganisms, and in this context by the microorganisms typical of breadmaking fermentation, to obtain a suitable volume and texture of the dough.

Lastly, "baking" denotes the process of converting fermented dough into bread by means of applying heat.

In this context, the present invention solves the technical problem associated with the incorporation of a sufficient amount of alpha-cyclodextrin [EFSA. Scientific Opinion on the substantiation of health claims related to alpha-cyclodextrin and reduction of post-prandial glycaemic responses (ID 2926, further assessment) pursuant to Article 13(1) of Regulation (EC). No 1924/2006. EFSA Journal 2012 10(6):2713] to achieve bread (preferably, conventionally processed bread, more preferably conventionally processed special bread) having a glycemic index that is low enough to be suitable for consumption by diabetics or pre-diabetics, and with physical and organoleptic properties that are acceptable and similar or identical to those of bread (preferably, conventionally processed bread, more preferably conventionally processed special bread) without alpha-cyclodextrin

The incorporation of alpha-cyclodextrin in the bread (preferably, conventionally processed bread, more preferably conventionally processed special bread) is not a trivial matter, since the addition causes undesirable effects on the dough and, subsequently, on the bread obtained, where it is necessary to adjust the remaining ingredients to achieve bread with acceptable organoleptic qualities.

The incorporation of alpha-cyclodextrin produces a lack of volume, ear, crust, and abnormal colouring, because this product absorbs water, reduces the water available for the formation of a suitable structure; in turn, it hinders the formation of the structure and the result is a decrease in gas trapped during fermentation (CO₂), with the subsequent penalization in the volume and the ear formation of the bread. The lack of water produces an abnormal colouring due to the advancement of the Maillard reaction.

As indicated above, the inventors of the present invention have unexpectedly and surprisingly seen that the addition of gluten to increase the total percentage of gluten both in the dough and in the final bread allows the aforementioned problems to be solved.

Therefore, to solve all these problems, in a first aspect, the present invention describes a preparation or formulation with a series of ingredients which, both at the qualitative and quantitative level, and always maintaining a conventional production process, allows bread to be obtained with the organoleptic parameters of conventional bread: conventional volume, crust, air hole formation, ear formation, colour and aroma and flavour.

The first aspect of the present invention, as indicated above, relates to a preparation of ingredients for producing bread comprising: alpha-cyclodextrin, wheat flour, and added gluten, characterised in that the amount of total gluten in the preparation of ingredients is at least 2.1% (in weight/weight, i.e., in g/100 g, hereinafter w/w).

The addition of a suitable amount of gluten is essential for being able to incorporate alpha-cyclodextrin. This molecule causes the effect whereby the dough becomes dense, leading to hard-to-chew bread without body and without volume.

As can be derived from the present specification, what is relevant is the amount of total gluten and it must be ensured that it reaches the values indicated in the present specification. The amount of added gluten will vary and will depend on the gluten that is already present in the rest of the ingredients or components of the preparation of ingredients. In any event, the preparation of ingredients of the present invention is preferably contemplated as comprising between 1% (w/w) and 3.5% (w/w) of added gluten, more preferably between 1.3% (w/w) and 2.6% (w/w), even more preferably between 1.35% (w/w) and 2.55% (w/w).

Preferably, the amount of alpha-cyclodextrin is at least 5 g of alpha-cyclodextrin per 50 g of starch present in the preparation of ingredients. The starch present in the preparation of ingredients commonly originates in its entirety from the flour used. Said flour used includes both the aforementioned wheat flour and any other type of flour that may comprise the preparation of ingredients or the dough or bread prepared from the preparation of ingredients.

The preparation of ingredients of the present invention is a preparation comprising all the ingredients necessary for preparing the bread with the exception of liquids (for example, water, more preferably drinking water).

As indicated above, the preparation of the present invention is for preparing dough and/or bread (preferably, conventionally processed bread, more preferably conventionally processed special bread), more preferably with a reduced glycemic index. Therefore, the preparation of ingredients can comprise any additional ingredient that is known and/or used now or in the future in the production of bread, preferably, conventionally processed bread (preferably, the preparation of ingredients of the present invention does not comprise chemical additives). Additionally, the preparation of ingredients of the present invention preferably does not comprise genetically modified organisms.

In a preferred embodiment, the amount of total gluten in the preparation of ingredients of the present invention is at least 2.2% (w/w), more preferably at least 2.25% (w/w).

In a more preferred embodiment, the amount of total gluten in the preparation of ingredients of the present invention is between 2.1% (w/w) and 4% (w/w), more preferably between 2.2% (w/w) and 3.8% (w/w), even more preferably between 2.25% (w/w) and 3.5% (w/w).

As indicated above, preferably, the amount of alpha-cyclodextrin is at least 5 g of alpha-cyclodextrin per 50 g of starch present in the preparation of ingredients of the present invention. More preferably, the amount of alpha-cyclodextrin in the preparation of ingredients of the present invention is at least 4% (w/w), more preferably at least 4.5% (w/w), more preferably between 4% (w/w) and 7% (w/w), even more preferably between 4.5% (w/w) and 6.5% (w/w).

In a more preferred embodiment, the preparation of ingredients of the present invention is for the production of conventionally processed bread (preferably conventionally processed special bread) with a reduced glycemic index and comprises 2.25% (w/w) of total gluten and 4.65% (w/w) of alpha-cyclodextrin.

In another more preferred embodiment, the preparation of ingredients of the present invention is for the production of conventionally processed bread (preferably conventionally processed special bread) with a reduced glycemic index and comprises 2.60% (w/w) of total gluten and 5.52% (w/w) of alpha-cyclodextrin. In this case, the preparation of ingredients of the present invention is for preparing conventionally processed bread (preferably, conventionally processed special bread) of the Pullman bread type.

In another embodiment, the preparation of ingredients of the present invention is contemplated as comprising an amount of total gluten greater than 10%, preferably greater than 10.2%, with respect to the total weight of the preparation.

Preferably, in the preparation of ingredients of the present invention the amount of wheat flour is between 50% (w/w) and 90% (w/w), more preferably between 60% (w/w) and 80% (w/w).

In another embodiment, the preparation of ingredients of the present invention is contemplated as comprising at least one additional source of starch. Preferably, the at least one additional source of starch is at least another type of flour in addition to the wheat flour (for example, rye flour). In this sense, the at least one additional source of starch can be added in the preparation of ingredients of the present invention in addition to the wheat flour or as a partial substitution thereof. In one embodiment, the wheat flour is partially substituted, preferably up to by 15% of its weight, more preferably up to 10% of its weight, with the at least one additional source of starch, necessarily incorporating a higher amount of added gluten to compensate for the lack of total gluten present in the preparation (due to the decrease in the amount of wheat flour).

In a preferred embodiment, the wheat flour used has a strength of between 140 and 350 W. The use of wheat flour with a strength of between 140 and 350 W or combinations of wheat flours each with a strength of between 140 and 350 W is contemplated.

In a preferred embodiment, the preparation of the invention also comprises sourdough starter, preferably up to 30% (w/w) with respect to the weight of the preparation of the invention, more preferably between 2% (w/w) and 25% (w/w), even more preferably between 2.2% (w/w) and 21% (w/w).

The use of sourdough starter contributes not only to an organoleptic improvement, but also directly affects the chewiness and durability of the bread produced from this preparation.

Therefore, the preparation of ingredients of the present invention is contemplated as consisting essentially of alpha-cyclodextrin, wheat flour, and added gluten, with the wheat flour, the alpha-cyclodextrin, the added gluten, and the total gluten being as indicated above. In another embodiment, the preparation of ingredients of the present invention is contemplated as consisting essentially of alpha-cyclodextrin, wheat flour, sourdough starter and added gluten, with the wheat flour, the alpha-cyclodextrin, the added gluten, the sourdough starter, and the total gluten being as indicated above. As can be derived, in these embodiments these would be the essential components or ingredients of the preparation of ingredients of the present invention, but the preparation of ingredients of the present invention can also comprise other ingredients characteristic of bread.

In another preferred embodiment, the preparation of the invention comprises between 0.9 and 3% (w/w) of salt, preferably between 1.2 and 1.8% (w/w) of salt, more preferably between 1.3% (w/w) and 1.8% (w/w) of salt, even more preferably between 1.38% (w/w) and 1.75% (w/w). This component allows imparting a structure to the bread and preventing the lack of consistency and volume.

The preparation of ingredients of the present invention also preferably does not comprise chemical additives.

Additionally, as indicated above, the preparation of ingredients of the present invention does not comprise genetically modified organisms.

In another particular embodiment, the preparation of ingredients of the present invention comprises:
- Wheat flour: between 77.5 and 87.0% (w/w).
- Alpha-cyclodextrin: 4.3 - 7.3% (w/w), preferably 5.5 - 7.3% (w/w).
- Added gluten: 1.7 - 3.5% (w/w).
- Rye flour: 0 - 8% (w/w).
- Up to 14.5% (w/w) of other components, such as yeast, salt, bread improver, or combinations thereof; or other sources of fibre; more preferably up to 4% (w/w), even more preferably up to 3.5% (w/w) of other components.

In this embodiment, the preparation of ingredients of the present invention preferably does not comprise sourdough starter.

The preparation of ingredients of the present invention is contemplated as consisting of:
- Wheat flour: Between 77.5 and 87.0% (w/w).
- Alpha-cyclodextrin: 4.3 - 7.3% (w/w), preferably 5.5 - 7.3% (w/w).
- Added gluten: 1.7 - 3.5% (w/w).
- Rye flour: 0-8% (w/w).
- Yeast: up to 14.5% (w/w), more preferably up to 4% (w/w), more preferably up to 3.5% (w/w), more preferred up to 1.75% (w/w), even more preferably 1.75% (w/w).
- Salt: up to 14.5% (w/w), more preferably up to 4% (w/w), more preferably up to 3.5% (w/w), more preferred up to 1.75% (w/w), even more preferably 1.75% (w/w).
- Bread improver: up to 14.5% (w/w), more preferably up to 4% (w/w), more preferably up to 3.5% (w/w), more preferred up to 1% (w/w), more preferably up to 0.54% (w/w), even more preferably 0.54% (w/w).

In a preferred embodiment, the preparation of the invention comprises:
- 75 - 85% (w/w) of wheat flour, preferably about 80% (w/w) of wheat flour, even more preferably 79.76% (w/w).
- 7.5 - 8.5% (w/w) of rye bran, more preferably about 8%, even more preferably 8% (w/w).
- 5.5 - 6.5% (w/w) of alpha-cyclodextrin, preferably about 6% (w/w), even more preferably 6.14% (w/w) of alpha-cyclodextrin.
- 1.5 - 2.5% (w/w) of added gluten, preferably about 2% (w/w), even more preferably 2.07% (w/w).
- Up to 10.5% (w/w) of other components, such as yeast, salt, bread improver, or other sources of fibre (more preferably, yeast, salt, bread improver, or combinations thereof), more preferably up to 4% (w/w), even more preferably up to 3.5% (w/w).

In this embodiment, preferably, the preparation of ingredients of the present invention does not comprise sourdough starter.

The preparation of the invention also preferably consists of:
- 75 - 85% (w/w) of wheat flour, preferably about 80% (w/w) of wheat flour, even more preferably 79.76% (w/w)
- 7.5 - 8.5% (w/w) of rye bran, more preferably about 8%, even more preferably 8% (w/w)
- 5.5 - 6.5% (w/w) of alpha-cyclodextrin, preferably about 6% (w/w), even more preferably 6.14% (w/w) of alpha-cyclodextrin
- 1.5 - 2.5% (w/w) of added gluten, preferably about 2% (w/w), even more preferably 2.07% (w/w).
- Up to 10.5% (w/w) of yeast, more preferably up to 4% (w/w), more preferably up to 3.5% (w/w), more preferred up to 1.75% (w/w), even more preferably 1.75% (w/w).
- Up to 10.5% (w/w) of salt, more preferably up to 4% (w/w), more preferably up to 3.5% (w/w), more preferred up to 1.75% (w/w), even more preferably 1.75% (w/w).
- Up to 10.5% (w/w) of bread improver, more preferably up to 4% (w/w), more preferably up to 3.5% (w/w), more preferred up to 1% (w/w), more preferably up to 0.54% (w/w), even more preferably 0.54% (w/w).

The preparation of the invention is contemplated as having the following composition:
- Wheat flour: Between 63.7 and 69.0% (w/w)
- Alpha-cyclodextrin: 4.3 - 5.8% (w/w), preferably 5.2 - 5.8% (w/w)
- Sourdough starter: 19 - 30% (w/w)
- Added gluten: 1.4 - 2.6% (w/w)
- Other sources of starch: 0 - 6.4% (w/w)
- Up to 9.40% (w/w) of other components, such as yeast, salt, bread improver, or other sources of fibre.

In one embodiment, the preparation of the invention consists of:
- 60 - 70% (w/w), preferably about 65% (w/w) of flour (preferably, wheat flour).
- 6 - 7% (w/w), preferably about 6.5% (w/w) of rye bran.
- 19 - 30% (w/w) of sourdough starter, preferably 19-20% (w/w) of sourdough starter.
- 4.5 - 5.5% (w/w), preferably about 5% (w/w) of alpha cyclodextrin.
- 1.5 - 2% (w/w), preferably about 1.6% (w/w) of gluten.
- Up to 9% (w/w) of other components, such as yeast, salt, bread improver, or other sources of fibre.

In another more preferred embodiment, the preparation of ingredients of the present invention comprises:
- Wheat flour: between 60% (w/w) and 85.0% (w/w), more preferably between 63% (w/w) and 80% (w/w).
- Alpha-cyclodextrin: between 4% (w/w) and 6% (w/w), more preferably between 4.5% (w/w) and 5.8% (w/w).
- Sourdough starter: between 2% (w/w) and 25% (w/w), more preferably between 2.2% (w/w) and 21% (w/w).
- Added gluten: between 1% (w/w) and 3% (w/w), more preferably between 1.3% (w/w) and 2.6% (w/w).
- Up to 5% (w/w) of at least one additional component, more preferably, up to 4% (w/w). The at least one additional component is yeast, salt, bread improver, or combinations thereof.

In this embodiment, the preparation of ingredients of the present invention preferably further comprises at least one additional source of starch (wheat flour is already a source of starch, therefore it refers to at least one additional source of starch): preferably, rye flour, rye bran, or combination thereof, more preferably rye bran. Preferably, the at least one additional source of starch is in an amount of up to 10% (w/w), more preferably up to 8% (w/w).

In another preferred embodiment, the preparation of ingredients of the present invention consists of:
- Wheat flour: between 60% (w/w) and 85.0% (w/w), more preferably between 63% (w/w) and 80% (w/w).
- Alpha-cyclodextrin: between 4% (w/w) and 6% (w/w), more preferably between 4.5% (w/w) and 5.8% (w/w).
- Sourdough starter: between 2% (w/w) and 25% (w/w), more preferably between 2.2% (w/w) and 21% (w/w).
- Added gluten: between 1% (w/w) and 3% (w/w), more preferably between 1.3% (w/w) and 2.6% (w/w).
- Up to 5% (w/w) of salt, more preferably up to 4% (w/w), more preferably up to 3% (w/w), more preferred up to 2% (w/w), even more preferably up to 1.6% (w/w).
- Up to 5% (w/w) of yeast, more preferably up to 4% (w/w), more preferably up to 3% (w/w), more preferred up to 2% (w/w), even more preferably up to 1.6% (w/w).
- Up to 5% (w/w) of bread improver, more preferably up to 4% (w/w), more preferably up to 3% (w/w), more preferred up to 2% (w/w), more preferably up to 1% (w/w), even more preferably up to 0.5% (w/w).

In another preferred embodiment, the preparation of ingredients of the present invention consists of:
- Wheat flour: between 60% (w/w) and 85.0% (w/w), more preferably between 63% (w/w) and 80% (w/w).
- Alpha-cyclodextrin: between 4% (w/w) and 6% (w/w), more preferably between 4.5% (w/w) and 5.8% (w/w).
- Sourdough starter: between 2% (w/w) and 25% (w/w), more preferably between 2.2% (w/w) and 21% (w/w).
- Added gluten: between 1% (w/w) and 3% (w/w), more preferably between 1.3% (w/w) and 2.6% (w/w).
- Up to 5% (w/w) of salt, more preferably up to 4% (w/w), more preferably up to 3% (w/w), more preferred up to 2% (w/w), even more preferably up to 1.6% (w/w).
- Up to 5% (w/w) of yeast, more preferably up to 4% (w/w), more preferably up to 3% (w/w), more preferred up to 2% (w/w), even more preferably up to 1.6% (w/w).
- Up to 5% (w/w) of bread improver, more preferably up to 4% (w/w), more preferably up to 3% (w/w), more preferred up to 2% (w/w), more preferably up to 1% (w/w), even more preferably up to 0.5% (w/w).
- At least one additional source of starch: preferably, rye flour, rye bran, or combination thereof, more preferably rye bran. Preferably, the at least one additional source of starch is in an amount of up to 10% (w/w), more preferably up to 8% (w/w).

The addition of gluten in order to reach a certain amount of total gluten in the preparation of ingredients allows said preparation of ingredients to enable obtaining breads obtained by conventional processing comprising suitable amounts of alpha-cyclodextrin and, therefore, a reduced glycemic index; and having suitable and even improved physical and organoleptic characteristics (as can be derived directly from the examples or tests included in the present specification).

As indicated above, in a second aspect, the present invention relates to dough obtained from the preparation of ingredients of the present invention.

"Dough" and the plural form thereof is understood to mean the product resulting from the kneading and fermentation of a mixture comprising wheat flour (or, like in the present case, a preparation of ingredients) and water (preferably drinking water).

The dough of the present invention is obtained from adding at least one liquid to the preparation of ingredients of the present invention. Therefore, the dough of the present invention comprises the preparation of ingredients of the present invention and at least one liquid, more preferably the dough of the present invention consists of the preparation of ingredients of the present invention and at least one liquid.

The at least one liquid is preferably water, more preferably drinking water. The dough of the present invention is contemplated as being able to comprise, along with water (preferably drinking water), additional liquids, preferably oil (preferably olive oil), vinegar, or combinations thereof. Therefore, in another preferred embodiment the at least one liquid is water (preferably drinking water), oil (preferably olive oil), and vinegar.

Preferably, the dough of the present invention comprises between 25% (w/w) and 50% (w/w) of the at least one liquid, more preferably between 30% (w/w) and 45% (w/w).

When the at least one liquid is water (preferably drinking water), the dough preferably comprises between 30 and 40% (w/w) of water, more preferably between 32% (w/w) and 36% (w/w) of water.

When the at least one liquid is water (preferably drinking water), oil (preferably olive oil), and vinegar, said liquids are in a combined total amount of between 30% (w/w) and 45% (w/w), more preferably between 35% (w/w) and 43% (w/w).

The dough of the present invention also preferably comprises between 50% (w/w) and 75% (w/w) of the preparation of the present invention, more preferably between 55% (w/w) and 70% (w/w).

As can be derived from that indicated above, the dough of the present invention is for the preparation, preferably, of conventionally processed bread (preferably conventionally processed special bread).

The dough of the present invention is also preferably for preparing dough and/or bread (preferably, conventionally processed bread, more preferably conventionally processed special bread) with a reduced glycemic index. Therefore, the dough of the present invention can comprise any additional ingredient that is known and/or used now or in the future in the production of bread, preferably, conventionally processed bread (preferably, the dough of the present invention does not comprise chemical additives).

The preparation of ingredients of the present invention and comprised in the dough of the present invention is exactly as explained above.

Therefore:
Preferably, the amount of alpha-cyclodextrin in the dough of the present invention is at least 5 g of alpha-cyclodextrin per 50 g of starch present in the dough of the present invention.

In a preferred embodiment, the amount of total gluten in the dough of the present invention is at least 1.3% (w/w), more preferably at least 1.4% (w/w).

In a more preferred embodiment, the amount of total gluten in the dough of the present invention is between 1.3% (w/w) and 4% (w/w), more preferably between 1.4% (w/w) and 3% (w/w), more preferably between 1.4% (w/w) and 2.5% (w/w), even more preferably between 1.46% (w/w) and 2.28% (w/w).

As indicated above, preferably, the amount of alpha-cyclodextrin is at least 5 g of alpha-cyclodextrin per 50 g of starch present in the dough of the present invention. More preferably, the amount of alpha-cyclodextrin in the dough of the present invention is at least 2.5% (w/w), more preferably at least 3% (w/w), more preferably between 2.5% (w/w) and 4.5% (w/w), even more preferably between 3% (w/w) and 4% (w/w).

As can be derived from the present specification, what is relevant is the amount of total gluten and it must be ensured that it reaches the values indicated in the present specification. The amount of added gluten will vary and will depend on the gluten that is already present in the rest of the ingredients or components of the dough. In any event, the dough of the present invention is preferably contemplated as comprising between 0.5% (w/w) and 3% (w/w) of added gluten, more preferably between 0.75% (w/w) and 2% (w/w), even more preferably between 0.9% (w/w) and 1.7% (w/w).

In a more preferred embodiment, the dough of the present invention is for the production of conventionally processed bread (preferably conventionally processed special bread) with a reduced glycemic index and comprises 1.46% (w/w) of total gluten and 3.01% (w/w) of alpha-cyclodextrin.

In another more preferred embodiment, the dough of the present invention is for the production of conventionally processed bread (preferably conventionally processed special bread) with a reduced glycemic index and comprises 1.61% (w/w) of total gluten and 3.42% (w/w) of alpha-cyclodextrin. In this case, the preparation of ingredients of the present invention is for preparing conventionally processed bread (preferably conventionally processed special bread) of the Pullman bread type.

Preferably, in the preparation of ingredients of the present invention the amount of wheat flour is between 35% (w/w) and 55% (w/w), more preferably between 40% (w/w) and 50% (w/w).

In a preferred embodiment, the dough of the present invention also comprises sourdough starter, preferably up to 20% (w/w) with respect to the weight of the dough of the present invention, more preferably between 1% (w/w) and 15% (w/w), even more preferably between 1.4% (w/w) and 14% (w/w).

The use of sourdough starter contributes not only to an organoleptic improvement, but also directly affects the chewiness and durability of the bread produced from this dough.

Therefore, the dough of the present invention is contemplated as consisting essentially of alpha-cyclodextrin, wheat flour, and added gluten, with the wheat flour, the alpha-cyclodextrin, the added gluten, and the total gluten being as indicated above. In another embodiment, the dough of the present invention is contemplated as consisting essentially of alpha-cyclodextrin, wheat flour, sourdough starter, and added gluten, with the wheat flour, the alpha-cyclodextrin, the added gluten, the sourdough starter, and the total gluten being as indicated above. As can be derived, in these embodiments these would be the essential components or ingredients of the dough of the present invention, but the dough of the present invention can also comprise other ingredients characteristic of bread.

In another embodiment, the invention is contemplated as relating to dough for producing breads comprising at least 5 g of alpha-cyclodextrin per 50 g of starch and at least 7.6% (w/w) of total gluten with respect to the weight of the dough, preferably between 7.6 and 8.2% (w/w).

In a preferred embodiment, the dough of the invention is obtained after adding water and kneading the preparation of the invention. In an even more preferred embodiment, between 45 and 65% (w/v) of water with respect to the total weight of the preparation of the invention, more preferably around 50% (w/v) of water with respect to the weight of the preparation will be added. By way of example, for 7 kg of preparation of the invention, about 3.5 litres of water will be added.

In another particular embodiment, the dough of the present invention comprises:
- Wheat flour: between 40% (w/w) and 60% (w/w), preferably between 45% (w/w) and 55% (w/w).
- Alpha-cyclodextrin: between 3% (w/w) and 5% (w/w), preferably between 3.5% (w/w) and 4% (w/w).
- Added gluten: between 0.75% (w/w) and 2.5% (w/w), preferably between 1% (w/w) and 2% (w/w).
- Rye flour: between 0% (w/w) and 5% (w/w).
- Up to 5% (w/w) of other components, such as yeast, salt, bread improver, or other sources of fibre (more preferably yeast, salt, bread improver, or combinations thereof), more preferably up to 4% (w/w), more preferably up to 3.5% (w/w), even more preferably up to 2.5% (w/w).
- Water: between 30% (w/w) and 45% (w/w), more preferably between 35% (w/w) and 40% (w/w).
- Oil (preferably olive oil): between 0.25% (w/w) and 1% (w/w).
- Vinegar: between 1 % (w/w) and 2% (w/w).

In this embodiment, the dough of the present invention preferably does not comprise sourdough starter.

Therefore, the dough of the present invention is contemplated as consisting of:
- Wheat flour: between 40% (w/w) and 60% (w/w), preferably between 45% (w/w) and 55% (w/w).
- Alpha-cyclodextrin: between 3% (w/w) and 5% (w/w), preferably between 3.5% (w/w) and 4% (w/w).
- Added gluten: between 0.75% (w/w) and 2.5% (w/w), preferably between 1% (w/w) and 2% (w/w).
- Rye flour: between 0% (w/w) and 5% (w/w).
- Water: between 30% (w/w) and 45% (w/w), more preferably between 35% (w/w) and 40% (w/w).
- Oil (preferably olive oil): between 0.25% (w/w) and 1% (w/w).
- Vinegar: between 1% (w/w) and 2% (w/w).
- Salt: up to 5% (w/w), more preferably up to 4% (w/w), more preferably up to 3% (w/w), even more preferably up to 1.5% (w/w).
- Yeast: up to 5% (w/w), more preferably up to 4% (w/w), more preferably up to 3% (w/w), even more preferably up to 1.5% (w/w).
- Bread improver: up to 5% (w/w), more preferably up to 4% (w/w), more preferably up to 3% (w/w), even more preferably up to 0.5% (w/w).

In another preferred embodiment, the dough of the present invention comprises:
- Wheat flour: between 40% (w/w) and 55% (w/w), preferably between 45% (w/w) and 50% (w/w), even more preferably 46.75% (w/w).
- Rye bran: between 3% (w/w) and 6% (w/w), even more preferably 4.7% (w/w).
- Alpha-cyclodextrin: between 3% (w/w) and 5% (w/w), preferably between 3.5% (w/w) and 4% (w/w), even more preferably 3.62% (w/w).
- Added gluten: between 0.75% (w/w) and 2.5% (w/w), preferably between 1% (w/w) and 2% (w/w), even more preferably 1.22% (w/w).
- Water: between 30% (w/w) and 45% (w/w), more preferably between 35% (w/w) and 40% (w/w), even more preferably 39.44% (w/w).
- Oil (preferably olive oil): between 0.25% (w/w) and 1% (w/w), even more preferably 0.47% (w/w).
- Vinegar: between 1% (w/w) and 2% (w/w), even more preferably 1.22% (w/w).
- Up to 5% (w/w) of other components, such as yeast, salt, bread improver, or other sources of fibre (more preferably, yeast, salt, bread improver, or combinations thereof), more preferably up to 4% (w/w), more preferably up to 3.5% (w/w), even more preferably up to 2.5% (w/w).

In this embodiment, the preparation of ingredients of the present invention preferably does not comprise sourdough starter.

The dough of the present invention also preferably consists of:
- Wheat flour: between 35% (w/w) and 55% (w/w), preferably between 45% (w/w) and 50% (w/w), even more preferably 46.75% (w/w).
- Rye bran: between 3% (w/w) and 6% (w/w), even more preferably 4.7% (w/w).
- Alpha-cyclodextrin: between 3% (w/w) and 5% (w/w), preferably between 3.5% (w/w) and 4% (w/w), even more preferably 3.62% (w/w).
- Added gluten: between 0.75% (w/w) and 2.5% (w/w), preferably between 1% (w/w) and 2% (w/w), even more preferably 1.22% (w/w).
- Water: between 30% (w/w) and 45% (w/w), more preferably between 35% (w/w) and 40% (w/w), even more preferably 39.44% (w/w).
- Oil (preferably olive oil): between 0.25% (w/w) and 1% (w/w), even more preferably 0.47% (w/w).
- Vinegar: between 1% (w/w) and 2% (w/w), even more preferably 1.22% (w/w).
- Salt: up to 5% (w/w), more preferably up to 4% (w/w), more preferably up to 3% (w/w), more preferably up to 1.5% (w/w), even more preferably 1.03% (w/w).
- Yeast: up to 5% (w/w), more preferably up to 4% (w/w), more preferably up to 3% (w/w), more preferably up to 1.5% (w/w), even more preferably 1.03% (w/w).
- Bread improver: up to 5% (w/w), more preferably up to 4% (w/w), more preferably up to 3% (w/w), even more preferably up to 0.5% (w/w), even more preferably 0.32% (w/w).

In another preferred embodiment, the dough of the present invention comprises:
- Wheat flour: between 30% (w/w) and 60% (w/w), more preferably between 40% (w/w) and 50% (w/w).
- Alpha-cyclodextrin: between 2.5% (w/w) and 4.5% (w/w), more preferably between 3% (w/w) and 4% (w/w).
- Sourdough starter: between 1% (w/w) and 15% (w/w), even more preferably between 1.4% (w/w) and 14% (w/w).

- Added gluten: between 0.5% (w/w) and 3% (w/w), more preferably between 1% (w/w) and 2% (w/w).
- Water: between 30% (w/w) and 40% (w/w).
- Up to 5% (w/w) of at least one additional component, more preferably, up to 4% (w/w). The at least one additional component is yeast, salt, bread improver, or combinations thereof.

In this embodiment, the preparation of ingredients of the present invention preferably further comprises at least one additional source of starch: preferably, rye flour, rye bran, or combination thereof, more preferably rye bran. Preferably, the at least one additional source of starch is in an amount of up to 7% (w/w), more preferably up to 5% (w/w).

In this embodiment, the dough is also contemplated as comprising additional liquids, preferably oil (preferably olive oil) and vinegar, each at a concentration of up to 3% (w/w), more preferably up to 1.5% (w/w).

The addition of gluten in order to reach a certain amount of total gluten in the dough allows said dough to enable obtaining breads obtained by conventional processing comprising suitable amounts of alpha-cyclodextrin and, therefore, a reduced glycemic index; and having suitable and even improved physical and organoleptic characteristics (as can be derived directly from the examples or tests included in the present specification).

Even more preferably, the method of kneading the preparation of ingredients of the present invention, which is also an object of this invention, uses a time of at least of 6 minutes, preferably between 6 and 12, more preferably around 10 minutes.

Preferably, the method of kneading comprises a prior pre-kneading for an approximate time of between 1 and 2 minutes.

In a preferred embodiment, the method of kneading comprises a pre-kneading of about 1 minute at a low speed and a kneading of about 5 minutes at a high speed.

In another preferred embodiment, suitable for kneading without sourdough starter, the method of kneading comprises a pre-kneading of about 2 minutes at a low speed and a kneading of about 8 minutes at a high speed.

Preferably, the low speed is at about 750 revolutions per minute, more preferably at 750 revolutions per minute.

Preferably, the high speed is at about 1500 revolutions per minute, more preferably at 1500 revolutions per minute.

By incorporating alpha-cyclodextrin, which is a soluble fibre, the dough acquires excessive tenacity, where reducing improver or the kneading time to achieve the desired rheology (extensibility) is not sufficient.

Moreover, the temperature must also be adjusted to prevent overheating and subsequent tearing of the dough. Preferably, the temperature used during kneading is comprised between 20 and 28.2°C.

In a particular embodiment, the dough of the invention is fermented dough. Preferably, the fermented dough is obtained by leaving the result of the kneading to rest for 10 - 30 minutes, preferably 20 minutes, and to ferment at a temperature of 25-32°C, preferably at 28°C, with a relative humidity of between 70 and 80%, preferably 78%, and a time of 30 to 120 minutes, preferably 70 minutes.

Frozen dough obtained by means of freezing the dough of the present invention, regardless of whether or not it has been fermented, is also an object of the invention.

In a particular embodiment, the frozen dough is obtained after having formed the piece which will subsequently be baked.

In a third aspect, the present invention relates to bread obtained using a preparation of ingredients or dough of the present invention.

Therefore, the bread of the present invention comprises at least 5 g of alpha-cyclodextrin per 50 g of starch.

In a preferred embodiment, the bread of the invention is special bread, preferably conventionally processed special bread.

In another preferred embodiment, the bread of the present invention is conventionally processed bread.

In another preferred embodiment, the bread of the invention is obtained by means of baking the dough of the present invention, more preferably by means of baking the dough of the present invention, or alternatively, a frozen dough obtained by means of freezing the dough of the present invention, at a temperature of between 160 and 240°C for 20 and 40 minutes.

As is known in the state of the art, when bread is baked about 30% of the water present in the dough used for the production of the bread is lost, more preferably when it is baked between 30% and 35% of the water present in the dough used for the production of the bread is lost. Therefore, taking this factor into account, the composition and concentration of the different components of the bread of the present invention can be derived directly from what has been indicated or explained above for the dough of the present invention and for the preparation of ingredients of the present invention.

As indicated above, the bread of the present invention has a reduced glycemic index and, therefore, is particularly suitable for consumption by the pre-diabetic and diabetic population. Additionally, the bread of the present invention has acceptable physical and organoleptic characteristics which are similar to, identical to, or better than those of bread which does not comprise alpha-cyclodextrin.

A fourth aspect of the present invention relates to prebaked bread from which the bread of the invention can be obtained. In particular, the invention relates to prebaked bread resulting from partially baking the dough of the present invention. In other words, the present invention also relates to prebaked bread obtained from the preparation of ingredients or from the dough of the present invention.

In a preferred embodiment, the prebaked bread of the present invention is special prebaked bread, preferably conventionally processed special prebaked bread.

Preferably, this prebaked bread is refrigerated or frozen immediately after partial baking, giving rise to refrigerated or frozen prebaked bread, which are also object of the invention.

As indicated above, the prebaked bread of the present invention has a reduced glycemic index and, therefore, is particularly suitable for consumption by the pre-diabetic and diabetic population. Additionally, the prebaked bread of the present invention has acceptable physical and organoleptic characteristics which are similar to, identical to, or better than those of bread which does not comprise alpha-cyclodextrin.

In a final aspect, the present invention relates to the use of gluten in combination with alpha-cyclodextrin for the preparation of bread with reduced glycemic index.

Preferably, the use of the present invention implies the use of added gluten for the purpose of reaching the necessary or required amounts of total gluten in the bread.

The alpha-cyclodextrin, the added gluten, the total gluten, and the bread with reduced glycemic index are exactly as explained above.

Bread with reduced glycemic index is bread of the present invention.

For better understanding, the present invention is described in detail below in reference to the following, non-limiting examples.

### Test 1

The following ingredients were incorporated in a kneading machine in the order in which they appear:
- 5 kg of wheat flour having a strength of W=140-220 (wheat flour with this strength has 9 grams of gluten per kg, and therefore 45 grams of gluten in total)
- 100 grams of salt
- 150 grams of compressed yeast (*Saccharomyces cerevisiae* was used in all the tests)
- 50 grams of bread improver
- 350 grams of alpha-cyclodextrin
- 3 litres of water

As derived from the foregoing, there is no added gluten in this case.

Therefore, the total gluten represents:
- 45 grams of the total 5650 grams of the preparation of ingredients, and therefore 0.796% (in g/100 g, weight/weight, i.e., w/w).
- 45 grams of the total 8650 grams of the dough, and therefore 0.52% (in g/100 g, weight/weight, i.e., w/w).

Once all the ingredients have been incorporated in the kneading machine, the steps listed below were followed:
- Kneading: Standard kneading for normal bread was performed. After a two-minute pre-kneading at a low speed to mix all the ingredients, a 10-minute kneading at a high speed was performed for the dough to acquire the desired elasticity and consistency. Temperature used in kneading: 25.3°C.
- Weighing: Weighing was performed by means of a weighing machine to form 80-g pieces of dough.
- Moulding: Moulding was performed in the dough rounder and it was allowed to rest for 20 minutes.
- Shaping the pieces: After said 20 minutes, the pieces were shaped, forming 80-gram dough balls.
- Fermentation: Once all the pieces were shaped, they were placed in tins suitable for use in bakery and introduced in the fermentation chamber with a temperature of about 35°C and an approximate humidity of 82%. The pieces were left to ferment until they doubled in volume (about 40 minutes).
- Baking: The fermented dough was introduced in the oven once preheated to a temperature of about 180°C and was baked for 18 minutes.

Observations and evaluation of this test:
- The dough is not very extensible at the end of kneading. The dough tore when its extensibility is checked using a windowpane test. This is due to the high tenacity that the dough acquires as a result of the incorporation of alpha-cyclodextrin, as a soluble fibre and an element that is unknown in common bread-making.
- At the end of the fermentation period, the bread did not develop normally but was "flattened", i.e., undeveloped, in the fermentation tins.
- Once fermented and after baking, the following was verified: Volume: The volume was much lower than expected and desired. Ear: Ear formation in the bread was negligible (ear-free bread). Crust: The crust was not uniform, nor was there a significant amount of it.
- Organoleptic characteristics:
   ∘ Colour: The resulting colour was very reddish and with even darker grains in the crumb and crust. It exhibited an irregular character.
   ∘ Air hole formation: Air hole formation was virtually inexistent (dense bread).
   ∘ Elasticity: Following manual deformation, it was verified that the bread did not recover at all.
   ∘ Chewiness: Very rubbery when chewed.
   ∘ Swallowing: Excessive effort when trying to swallow it.

Overall, the results of this test were not acceptable.

### Test 2

The following ingredients were incorporated in a kneading machine in the order in which they appear:
- 5 kg of wheat flour having a strength of W=140-220 (wheat flour with this strength has 9 grams of gluten per kg, and therefore 45 grams of gluten in total)
- 350 grams of alpha-cyclodextrin
- 30 grams of bread improver
- 100 grams of yeast
- 3 litres of water
- 100 grams of salt

As derived from the foregoing, there is no added gluten in this case.

Therefore, the total gluten represents:
- 45 grams of the total 5580 grams of the preparation of ingredients, and therefore 0.806% (in g/100 g, weight/weight, i.e., w/w).
- 45 grams of the total 8580 grams of the dough, and therefore 0.52% (in g/100 g, weight/weight, i.e., w/w).

Once all the ingredients have been incorporated in the kneading machine, the steps listed below were followed:
- Kneading: After a one-minute pre-kneading at a low speed to mix all the ingredients, to prevent excessive kneading and overheating of the dough, an 8-minute kneading at a high speed was performed for the dough to acquire the desired elasticity and consistency. It was observed that the dough was not fine enough.
   Temperature used in kneading: 25.3°C.
- Weighing: Weighing was performed by means of a weighing machine to form 80-g and 110-g pieces of dough.
- Moulding: Moulding was performed in the dough rounder and it was allowed to rest for 20 minutes.
- Shaping the pieces: After said 20 minutes, the pieces were shaped, forming 80-gram and 110-gram dough balls.
- Fermentation: Once all the pieces were shaped, they were placed in tins suitable for use in bakery and introduced in the fermentation chamber with a temperature of about 32°C and an approximate humidity of 78%. The pieces were left to ferment until they doubled in volume (about 50 minutes).
- Baking: The fermented dough was introduced in the oven once preheated to a temperature of about 180°C and was baked for about 18 minutes.

Observations and evaluation of this test:
- The dough was not extensible enough at the end of kneading. The dough tore when its extensibility is checked using a windowpane test.
- At the end of the fermentation period, the bread did not develop normally but was "flattened", i.e., undeveloped, in the fermentation tins.
- Once fermented and after baking, no differences were observed with respect to the bread obtained in test 1: Volume: The volume was much lower than expected and desired. Ear: Ear formation in the bread was negligible (ear-free bread). Crust: The crust was not uniform, nor was there a significant amount of it.
- Organoleptic characteristics:
   ∘ No significant changes were observed at the level of colour, air hole formation, elasticity, chewiness, and swallowing with respect to test 1. Overall, the results of this test were not acceptable.

### Test 3

The following ingredients were incorporated in a kneading machine in the order in which they appear:
- 5 kg of strong wheat flour (wheat flour with this strength has 12 grams of gluten per kg, and therefore 60 grams of gluten in total)
- 350 g of alpha-cyclodextrin
- 30 g of bread improver
- 50 g of added gluten
- 100 g of compressed yeast
- 3 litres of water
- 100 g of salt, previously diluted in lukewarm water

Therefore, the total gluten represents:
- 110 grams of the total 5630 grams of the preparation of ingredients, and therefore 1.95% (in g/100 g, weight/weight, i.e., w/w).
- 110 grams of the total 8630 grams of the dough, and therefore 1.27% (in g/100 g, weight/weight, i.e., w/w).

Once all the ingredients have been incorporated in the kneading machine, the steps listed below were followed:
- Kneading: After a one-minute pre-kneading at a low speed to mix all the ingredients, a 6-minute kneading at a high speed was performed to prevent excessive kneading and overheating of the dough. Temperature used in kneading: 25.3°C.
- Weighing: Weighing was performed by means of a weighing machine to form 80-g and 110-g pieces of dough.
- Moulding: Moulding was performed in the dough rounder and it was allowed to rest for 20 minutes.
- Shaping the pieces: After said 20 minutes, the pieces were shaped, forming 80-gram and 110-gram dough balls.
- Fermentation: Once all the pieces were shaped, they were placed in tins suitable for use in bakery and introduced in the fermentation chamber with a temperature of about 32°C and an approximate humidity of 78%. The pieces were left to ferment until they doubled in volume (about 70 minutes).
- Baking: The fermented dough was introduced in the oven once preheated to a temperature of about 180°C and was baked for about 18 minutes.

Observations and evaluation of this test:
- A greater tolerance of the dough when its extensibility was checked using a windowpane test was observed, although it was not sufficient.
- At the end of the fermentation period, the bread did not have an acceptable appearance. Again, it was "flattened", i.e., undeveloped, in the fermentation tins.
- Once fermented and after baking, no differences were observed with respect to the bread obtained in test 1:
- Organoleptic characteristics:
   ∘ No significant changes were observed at the level of colour, air hole formation, elasticity, chewiness, and swallowing with respect to test 1.

Overall, the results of this test were not acceptable.

### Test 4

The following ingredients were incorporated in a kneading machine in the order in which they appear:
- 2.5 kg of wheat flour having a strength of W=140-220 (wheat flour with this strength has 9 grams of gluten per kg, and therefore 22.5 grams of gluten in total)
- 2.5 kg of strong wheat flour (wheat flour with this strength has 12 grams of gluten per kg, and therefore 30 grams of gluten in total)
- 3.5 litres of water
- 100 g of salt
- 100 g of compressed yeast
- 1 kg of sourdough starter (represents or includes 6 grams of gluten)
- 30 grams of bread improver
- 400 grams of alpha-cyclodextrin
- 50 g of added gluten

Therefore, the total gluten represents:
- 108.5 grams of the total 6680 grams of the preparation of ingredients, and therefore 1.62% (in g/100 g, weight/weight, i.e., w/w).
- 108.5 grams of the total 10180 grams of the dough, and therefore 1.07% (in g/100 g, weight/weight, i.e., w/w).

### Production of sourdough starter as an intermediate ingredient:

### Ingredients:

- 1 kg of active starter (initial sourdough starter culture)
- 1 kg of wheat flour having a strength of W=300-350
- 0.5 litres of water
- 10 g salt
- 5 g of compressed yeast

Process:
The process began with an active or initial sourdough starter originating from the kneading of flour and water, and successive "refreshing" every 8 hours (feeding the microbial flora with successive incorporations of flour and water every 8 hours and fermentations at room temperature and in refrigeration, performed for the last 2 years).

The ingredients were incorporated in the kneading machine and kneading was performed at a low speed for 1 minute and at a high speed for 6 minutes.

The dough was removed and left to ferment in bulk for 8 hours at room temperature, except for the last refreshing which was kept in a fermentation chamber at a temperature of 4-5°C and ambient humidity.

Once all the ingredients have been incorporated in the kneading machine, the steps listed below were followed:
- Kneading: After a one-minute pre-kneading at a low speed to mix all the ingredients, to prevent excessive kneading and overheating of the dough, a 6-minute kneading at a high speed was performed for the dough to acquire the desired elasticity and consistency. Temperature used in kneading: 24.2°C.
- Weighing: Weighing was performed by means of a weighing machine to form 80-g and 110-g pieces of dough.
- Moulding: Moulding was performed in the dough rounder and it was allowed to rest for 20 minutes.
- Shaping the pieces: After said 20 minutes, the pieces were shaped, forming 80-gram and 110-gram dough balls.
- Fermentation: Once all the pieces were shaped, they were placed in tins suitable for use in bakery and introduced in the fermentation chamber with a temperature of about 28°C and an approximate humidity of 78%. The pieces were left to ferment until

they doubled in volume (about 70 minutes).
- Baking: The fermented dough was introduced in the oven once preheated to a temperature of about 180°C and was baked for about 18 minutes.

Observations and evaluation of this test:
- A sufficient degree of extensibility that is significantly better than in the preceding tests was observed in the dough.
- At the end of the fermentation period, the bread did not have an acceptable appearance. A lack of volume was observed.
- Once fermented and after baking, the following details were observed: Volume: The volume was slightly lower than desired. Ear: Ear formation in the bread was negligible (ear-free bread). Crust: The crust was not uniform, nor was there a significant amount of it.
- Organoleptic characteristics:
   ∘ Light golden colour. There were no spots on the crust.
   ∘ Air hole formation was somewhat greater than in the preceding tests and rather regular, but the air holes were lacking in size and irregularity.
   ∘ It had greater elasticity and better recovery. The structure of the pieces had greater consistency.
   ∘ Chewiness: Some improvement was observed, although it was not enough. The resulting bread was chewy, although not as much as in the preceding tests.
   ∘ Swallowing: Difficulty was perceived when swallowing the pieces. There were no apparent advancements.

Overall, the results of this test were not acceptable.

### Test 5

The following ingredients were incorporated in a kneading machine in the order in which they appear:
- 5 kg of strong wheat flour (wheat flour with this strength has 12 grams of gluten per kg, and therefore 60 grams of gluten in total)
- 3.5 litres of water
- 100 g of salt
- 100 g of compressed yeast
- 1.5 kg of sourdough starter (represents or includes 9 grams of gluten)
- 30 g of bread improver
- 420 g of alpha-cyclodextrin
- 100 g of added gluten

Therefore, the total gluten represents:
- 169 grams of the total 7250 grams of the preparation of ingredients, and therefore 2.33% (in g/100 g, weight/weight, i.e., w/w).
- 169 grams of the total 10750 grams of the dough, and therefore 1.57% (in g/100 g, weight/weight, i.e., w/w).

Once all the ingredients have been incorporated in the kneading machine, the steps listed below were followed:
- Kneading: After a one-minute pre-kneading at a low speed to mix all the ingredients, a 6-minute kneading at a high speed was performed for the dough to acquire the desired elasticity and consistency. Temperature used in kneading: 23.6°C.
- Weighing: Weighing was performed by means of a weighing machine to form 80-g and 110-g pieces of dough.
- Moulding: Moulding was performed in the dough rounder and it was allowed to rest for 20 minutes.
- Shaping the pieces: After said 20 minutes, the pieces were shaped, forming 80-gram and 110-gram dough balls.
- Fermentation: Once all the pieces were shaped, they were placed in tins suitable for use in bakery and introduced in the fermentation chamber with a temperature of about 28°C and an approximate humidity of 78%. The pieces were left to ferment until they doubled in volume (about 70 minutes).
- Baking: The fermented dough was introduced in the oven once preheated to a temperature of about 180°C and was baked for about 18 minutes.

Observations and evaluation of this test:
- An acceptable degree of extensibility significantly better than in the preceding tests was observed in the dough.
- At the end of the fermentation period, improvement in the volume of the pieces was observed, although it could be further improved.
- Once fermented and after baking, the following details were observed: Volume: More developed volume, slightly lower than desired. Ear: Acceptable but not optimal. Crust: Acceptable but not optimal.
- Organoleptic characteristics:
   ∘ A more golden colour was observed and the Maillard reaction was further enhanced, although not yet optimum. There were no spots.
   ∘ Air hole formation was somewhat greater than all the preceding tests, but the air holes were lacking in size and irregularity.
   ∘ It had much more elasticity and much better recovery because the structure of the pieces had greater consistency.
   ∘ A significant improvement in chewiness was observed, without being completely acceptable.
   ∘ In terms of chewiness, certain difficulty was perceived when swallowing the obtained pieces of bread, but a significant advancement was observed, without reaching the optimal state.

Overall, the results of this test were acceptable.

### Test 6

The following ingredients were incorporated in a kneading machine in the order in which they appear:
- ½ kg of white rye flour
- 5 kg of strong wheat flour (wheat flour with this strength has 12 grams of gluten per kg, and therefore 60 grams of gluten in total)
- 3.9 litres of water
- 110 g of salt
- 110 g of compressed yeast
- 1.5 kg of sourdough starter (represents or includes 9 grams of gluten)
- 34 g of bread improver
- 420 g of alpha-cyclodextrin
- 200 g of added gluten

Therefore, the total gluten represents:
- 269 grams of the total 7874 grams of the preparation of ingredients, and therefore 3.42% (in g/100 g, weight/weight, i.e., w/w).
- 269 grams of the total 11774 grams of the dough, and therefore 2.28% (in g/100 g, weight/weight, i.e., w/w).

Once all the ingredients have been incorporated in the kneading machine, the steps listed below were followed:
- Kneading: After a one-minute pre-kneading at a low speed to mix all the ingredients, a 5-minute kneading at a high speed was performed for the dough to acquire the desired elasticity and consistency. Temperature used in kneading: 24.2°C.
- Weighing: Weighing was performed by means of a weighing machine to form 80-g, 110-g, and 500-g of pieces of dough.
- Moulding: Moulding of the small pieces was performed in the dough rounder and they were allowed to rest for 20 minutes. The 500-g pieces were left to rest for 30 minutes after weighing and moulding.
- Shaping the pieces: After said 20 minutes, the pieces were shaped, forming 80-gram and 110-gram dough balls. In the case of the 500-g pieces, after 30 minutes of rest they were shaped into a loaf and again left to rest for about 90 minutes.
- Fermentation: Once formed, the small pieces were placed in tins suitable for use in bakery and introduced in the fermentation chamber with a temperature of about 28°C and an approximate humidity of 78%. They were left to ferment until they doubled in volume (about 70 minutes).

The ½ kg pieces were left to ferment on a tray at room temperature (26°C) and a humidity of 35% for a fermentation time of 120 minutes.
- Baking: The already fermented small pieces were introduced in the oven once preheated to a temperature of about 180°C and were baked for about 22 minutes. The 500-g loaves were baked in a wood fired oven previously heated to a temperature of about 220°C with a baking time of about 40 minutes.

Observations and evaluation of this test:
- An acceptable degree of extensibility was observed in the dough.
- A surprising improvement in the volume of the pieces, approximating the optimal level, was observed.
- After baking, the following details were observed:
   ∘ Volume: A significant improvement, approximating the optimal level, was observed in both types of pieces.
   ∘ Ear: Improved significantly but not optimal.
   ∘ Crust: Improved significantly but not optimal.
- Organoleptic characteristics:
   ∘ The intended colour was obtained, reaching its optimal level. The crusts on both the bread rolls and the loaves showed a pale reddish colour and a russet crumb colour.
   ∘ The air hole formation achieved was optimal in both types of breads, being irregular and very noticeable.
   ∘ The elasticity and recovery of both breads are optimal.
   ∘ The chewiness and swallowing of the resulting breads were considered optimal. Overall, the results of this test were acceptable.

### Test 7

The following ingredients were incorporated in a kneading machine in the order in which they appear:
- 1 kg of rye bran
- 10 kg of wheat flour having a strength of W=140-220 (wheat flour with this strength has 9 grams of gluten per kg, and therefore 90 grams of gluten in total)
- 8.4 litres of water
- 220 g of salt
- 220 g of yeast
- 3 kg of sourdough starter (represents or includes 18 grams of gluten)
- 68 g of bread improver
- 240 g of added gluten
- 720 g of alpha-cyclodextrin

Therefore, the total gluten represents:
- 348 grams of the total 15468 grams of the preparation of ingredients, and therefore 2.25% (in g/100 g, weight/weight, i.e., w/w).
- 348 grams of the total 23868 grams of the dough, and therefore 1.46% (in g/100 g, weight/weight, i.e., w/w).

Once all the ingredients have been incorporated in the kneading machine, the steps listed below were followed:
- Kneading: After a one-minute pre-kneading at a low speed to mix all the ingredients, to prevent excessive kneading and overheating of the dough, a 5-minute kneading at a high speed was performed for the dough to acquire the desired elasticity and consistency. Temperature used in kneading: 24°C.
- Weighing: Weighing was performed by means of a weighing machine to form 80-g, 110-g, 120-g, and 500-g pieces of dough, to make bread rolls, ciabatta bread, and loaves, respectively.
- Moulding: Moulding of the small pieces was performed in the dough rounder and they were allowed to rest for 20 minutes. The 500-g pieces were left to rest for 15 minutes after weighing and moulding. The 120-g pieces, intended for ciabatta bread, did not require moulding.
- Shaping the pieces: After said 20 minutes, the pieces were shaped, forming 80-gram and 110-gram dough balls. In the case of the 500-g pieces, after 15 minutes of rest they were shaped into a loaf and again left to rest for about 90 minutes to ferment. The pieces intended for ciabattas were extended and left to rest for 4 hours to ferment.
- Fermentation: Once formed, the small pieces were placed in tins suitable for use in bakery and introduced in the fermentation chamber with a temperature of about 28°C and an approximate humidity of 78%. They were left to ferment until they doubled in volume (about 70 minutes).

The ½ kg pieces and the 120-g pieces were left to ferment on a tray at room temperature (26°C) and a humidity of 35%, the 500-g pieces being left for a fermentation time of 90 minutes and the pieces intended for ciabatta being left for 4 hours at room temperature.
- Baking: Once fermented, the pieces intended for bread rolls (80 g and 110 g) were introduced in the oven once preheated to a temperature of about 180°C and were baked for about 22 minutes. The pieces for ciabatta bread were baked in the same conditions, but for 34-40 minutes. The 500-g loaves were baked in a wood fired oven previously heated to a temperature of about 220°C with a baking time of about 45 minutes.

Observations and evaluation of this test:
- The dough acquired the desired texture with respect to the desired tenacity and extensibility rheological parameters, said dough being considered optimal.
- All the pieces acquired the desired volume, reaching the optimal level.
- After baking, the following details were observed:
   ∘ Volume: The desired or optimal volume was observed in all the types of pieces that have been produced.
   ∘ Ear: The desired level was obtained, where it can be described as optimal.
   ∘ Crust: The desired crust was obtained, where it can be described as optimal.
- Organoleptic characteristics:
   ∘ The intended colour was obtained, reaching its optimal level. The crusts on both the bread rolls and the loaves showed a pale reddish colour and a russet crumb colour.
   ∘ The air hole formation achieved was optimal in all the types of breads, being irregular and very noticeable.
   ∘ The elasticity and recovery of all the breads obtained can be considered optimal. ∘ The chewiness and swallowing of the resulting breads were considered optimal.
   ∘ The smell of the bread was normal, but with very pleasant hints of rye.
   ∘ The taste was very pleasant. The mixture of flavours provided by the rye bran together with the lactic acid-acetic acid taste of the sourdough starter cause the bread to have a much more pleasant taste.

The results obtained in this test were surprisingly positive and superior.

### Test 8

The following ingredients were incorporated in a kneading machine in the order in which they appear:
- 1 kg of rye bran
- 10 kg of wheat flour having a strength of W=140-220 (wheat flour with this strength has 9 grams of gluten per kg, and therefore 90 grams of gluten in total)
- 8.4 litres of water
- 220 g of salt
- 220 g of yeast
- 68 g of bread improver
- 260 g of added gluten
- 260 ml of vinegar
- 100 ml of olive oil
- 770 g of alpha-cyclodextrin

Therefore, the total gluten represents:
- 350 grams of the total 12538 grams of the preparation of ingredients, and therefore 2.79% (in g/100 g, weight/weight, i.e., w/w).
- 350 grams of the total 21298 grams of the dough, and therefore 1.64% (in g/100 g, weight/weight, i.e., w/w).

Once all the ingredients have been incorporated in the kneading machine, the steps listed below were followed:
- Kneading: After a two-minute pre-kneading at a low speed to mix all the ingredients, to prevent excessive kneading and overheating of the dough, an 8-minute kneading at a high speed was performed for the dough to acquire the desired elasticity and consistency.

Temperature used in kneading: 24°C.
- Weighing: Weighing was performed by means of a weighing machine to form 110-g and 500-g pieces of dough.
- Rest: The pieces were left to rest for 20 minutes after weighing.
- Moulding: Moulding of the small pieces was performed in the dough rounder and they were allowed to rest for 20 minutes. The 500-g pieces were left to rest for 15 minutes after weighing and moulding.
- Shaping the pieces: 110-gram dough balls were formed. In the case of the 500-g pieces, they were shaped into a loaf and again left to rest for about 90 minutes.
- Fermentation: Once formed, the small pieces were placed in tins suitable for use in bakery and introduced in the fermentation chamber with a temperature of about 28°C and an approximate humidity of 78%. They were left to ferment until they doubled in volume (about 70-75 minutes).

The ½ kg pieces were left to ferment on a tray at room temperature (26°C) and a humidity of 35% for a fermentation time of 90 minutes.
- Baking: The already fermented small pieces were introduced in the oven once preheated to a temperature of about 170°C and were baked for about 34-50 minutes at 180°C. The 500-g loaves were baked in a wood fired oven previously heated to a temperature of about 210°C with a baking time of about 45 minutes.

Observations and evaluation of this test:
- Kneading was performed for two minutes at a low speed and eight minutes at a high speed. Despite increasing the kneading time, the dough acquired the desired texture with respect to the desired tenacity and extensibility rheological parameters, the process being considered optimal.
- With respect to fermentation, all the pieces reached the desired volume, this step being considered optimal.
- After baking, the following details were observed:
   ∘ Volume: The desired or optimal volume was observed in all the pieces that have been produced.
   ∘ Ear: Certain lack of ear was observed, although it was described as optimal.
   ∘ Crust: Very fine and even crust was obtained, where it can be described as optimal.
- Organoleptic characteristics:
   ∘ The intended colour was obtained, reaching its optimal level. The crusts on both the bread rolls and the loaves showed a pale reddish colour and a russet crumb colour.
   ∘ The air hole formation achieved was optimal in all the types of breads, being irregular and very noticeable.
   ∘ The elasticity and recovery of all the breads obtained can be considered optimal.
   ∘ The chewiness and swallowing of the resulting breads were considered optimal.
   ∘ The smell of the bread was normal, but with very pleasant hints of rye.
   ∘ The taste was very pleasant. The mixture of flavours provided by the rye bran together with the lactic acid-acetic acid taste of the sourdough starter cause the bread to have a much more pleasant taste.

Overall, the results of this test were acceptable.

### Test 9

In this case, Pullman bread was prepared with alpha-cyclodextrin.

The following ingredients were incorporated in a kneading machine in the order in which they appear:
- 250 g of rye bran (chaffs)
- 2.5 kg of wheat flour having a strength of W=140-220 (flour with this strength has 9 grams of gluten per kg, and therefore 22.5 grams of gluten in total)
- 50 g of salt
- 50 g of yeast
- 12.5 g of Conserval 0080AC (natural improver obtained from hawthorn berry)
- 35 g of propionic sourdough starter ("devitalized sourdough starter")
- 37.5 g of devitalized wheat sourdough starter
- 60 g of added gluten
- 75 g of vinegar containing 6% acetic acid
- 75 g of olive oil
- 175 g of alpha-cyclodextrin
- 1.8 litres of water

Therefore, the total gluten represents:
- 82.42 grams of the total 3170 grams of the preparation of ingredients, and therefore 2.60% (in g/100 g, weight/weight, i.e., w/w).
- 82.42 grams of the total 5120 grams of the dough, and therefore 1.61% (in g/100 g, weight/weight, i.e., w/w).

All the already weighed solid elements were introduced in the kneading machine (except the yeast which was added 5 minutes before the end of kneading). They were mixed for 2 minutes so that a good mixing is assured. All the liquid ingredients were then added.

Once all the ingredients have been incorporated in the kneading machine, the steps listed below were followed:
- Kneading: After a two-minute pre-kneading at a low speed (750 revolutions per minute - revolutions of the motor; a characteristic that is also applicable to tests 1 to 8) to mix all the ingredients, thereby preventing the excess kneading and overheating of the dough, a 7-minute kneading at a high speed (1500 revolutions per minute - revolutions of the motor; a characteristic that is also applicable to tests 1 to 8) was performed for the dough to acquire the desired elasticity and consistency. The pH of the dough was 5.4. Temperature used in kneading: 24°C. Rest in bulk: 15 minutes
- Weighing: Weighing was performed by means of a weighing machine to form 700-g pieces of dough.
- Moulding: The moulding of the pieces was performed in the dough rounder and they were allowed to rest for 20 minutes after weighing and moulding.
- Shaping the pieces: After said 20 minutes, the pieces were shaped into a rectangular prism shape (typical shape of Pullman bread). Once formed, they were transferred to the pans.
- Fermentation: Once the pieces have been introduced in the pans and the lids of the pans put in place, they were introduced in the fermentation chamber with a temperature of about 35°C and an approximate humidity of 82%. They were left to ferment until they doubled in volume (about 130 minutes).
- Baking: Once the pieces inside the pans have been fermented, baking followed. The oven (rack oven) was pre-heated to a temperature of 180°C for about 34 minutes.

Observations and evaluation of this test:
- The dough acquired the desired texture with respect to the desired tenacity and extensibility rheological parameters, these parameters being considered optimal.
- The pieces acquired the desired volume, reaching the optimal level.
- After baking, the following details were observed:
   ∘ Volume: The desired or optimal volume was observed.
   ∘ Crust: The desired crust was obtained, where it can be described as optimal.
- Organoleptic characteristics:
   ∘ The intended colour was obtained, reaching its optimal level.
   ∘ The air hole formation achieved was optimal. Taking into account the well-known fact that Pullman bread exhibits almost zero air hole formation, this Pullman bread which has been attained therefore exhibits a significant air hole formation with respect to what is known in the state of the art (more similar to the conventional bread for daily consumption), giving a taste of homemade bread, different from the conventionally known Pullman bread (giving it an additional point of characteristic flavour and being differentiated from the rest); said air hole formation being irregular and very noticeable.
   ∘ The elasticity and recovery obtained were optimal.
   ∘ The chewiness and swallowing of the resulting bread were considered optimal.
   ∘ The smell of the bread was normal, but with very pleasant hints provided by the sourdough starter and the rye bran.
   ∘ The taste was very pleasant. The mixture of flavours provided by the rye bran together with the lactic acid-acetic acid taste of the sourdough starter cause the bread to have a much more pleasant taste.

The results obtained in this test were surprisingly positive and superior.

### Conclusions:

The addition of alpha-cyclodextrin as an ingredient in the production of bread presents various problems the solution of which was not evident as demonstrated by the host of tests performed and the complexity in producing same.

Lastly, these problems have been solved with the suitable choice of ingredients, more specifically with the addition of gluten to obtain the amount of total gluten needed to avoid the negative effects of alpha-cyclodextrin in breadmaking and to thereby enable obtaining breads by means of conventional processing with the physical and organoleptic characteristics typical of bread which is produced by means of conventional processing and does not comprise alpha-cyclodextrin.

Additionally, as demonstrated in Test 9, Pullman bread with improved physical and organoleptic characteristics can be obtained with the present invention.

Therefore, the present invention allows obtaining breads with a high organoleptic quality which exhibit physical characteristics that have reached an optimal level and are fully accepted by all the members of the tasting panel evaluating the tests.

## Claims

1. A preparation of ingredients for producing bread comprising alpha-cyclodextrin, wheat flour, and added gluten, **characterised in that** the amount of total gluten in the preparation of ingredients is at least 2.1% (w/w).

2. A preparation of ingredients according to claim 1 **characterised in that** the amount of alpha-cyclodextrin is at least 5 g of alpha-cyclodextrin per 50 g of starch present in the preparation of ingredients.

3. The preparation of ingredients according to claim 1 or 2, **characterised in that** the amount of total gluten is between 2.1% (w/w) and 4% (w/w).

4. The preparation of ingredients according to claim 3, **characterised in that** the amount of total gluten is between 2.25% (w/w) and 3.5% (w/w).

5. The preparation of ingredients according to any of claims 1 to 4, **characterised in that** the amount of alpha-cyclodextrin is at least 4% (w/w).

6. The preparation of ingredients according to any of claims 1 to 5, **characterised in that** the amount of alpha-cyclodextrin is between 4.5% (w/w) and 6.5% (w/w).

7. The preparation of ingredients according to any of claims 1 to 6, **characterised in that** the wheat flour used has a strength of between 140 and 350 W.

8. The preparation of ingredients according to any of claims 1 to 7, **characterised in that** it further comprises sourdough starter.

9. The preparation of ingredients according to claim 8, **characterised in that** the sourdough starter is in an amount of between 2% (w/w) and 25% (w/w).

10. The preparation of ingredients according to claim 8 or 9, **characterised in that** the sourdough starter is in an amount of between 2.2% (w/w) and 21% (w/w).

11. The preparation of ingredients according to any of claims 1 to 10, **characterised in that** it comprises between 0.9% (w/w) and 3% (w/w) of salt.

12. The preparation of ingredients according to any of the preceding claims comprising:
• wheat flour: between 77.5 and 87.0% (w/w)
• alpha-cyclodextrin: 4.3 - 7.3% (w/w), preferably 5.5 - 7.3% (w/w)
• added gluten: 1.7-3.5% (w/w)
• rye flour: 0-8% (w/w)
• up to 14.5% (w/w) of yeast, salt, bread improver, or combinations thereof.

13. The preparation of ingredients according to any of claims 1 to 11, **characterised in that** it comprises:
• 75 - 85% (w/w) of wheat flour
• 7.5 - 8.5% (w/w) of rye bran
• 5.5 - 6.5% (w/w) of alpha-cyclodextrin
• 1.5 - 2.5% (w/w) of added gluten
• up to 10.5% (w/w) of yeast, salt, bread improver, or combinations thereof.

14. The preparation of ingredients according to claim 12 or 13, **characterised in that** it does not comprise sourdough starter.

15. The preparation of ingredients according to any of claims 1 to 11 comprising:
• wheat flour: between 60% (w/w) and 85.0% (w/w)
• alpha-cyclodextrin: between 4% (w/w) and 6% (w/w)
• sourdough starter: between 2% (w/w) and 25% (w/w)
• added gluten: between 1% (w/w) and 3% (w/w)
• up to 5% (w/w) of yeast, salt, bread improver, or combinations thereof.

16. The preparation of ingredients according to claim 15, **characterised in that** it comprises at least one additional source of starch.

17. The preparation of ingredients according to claim 16, **characterised in that** at least an additional source of starch is rye flour, rye bran, or combination thereof.

18. The preparation of ingredients according to claim 16 or 17, **characterised in that** at least one additional source of starch is in an amount of up to 10% (w/w).

19. The dough obtained from a preparation of ingredients according to any of claims 1 to 18.

20. The dough **characterised in that** it comprises at least one liquid and the preparation of ingredients according to any of claims 1 to 18, wherein at least the liquid is water.

21. The dough according to claim 21, **characterised in that** at least one liquid is present in an amount of between 25% (w/w) and 50% (w/w).

22. The dough according to claim 20 or 21, **characterised in that** at least one liquid is water.

23. The dough according to claim 20 or 21, **characterised in that** the at least one liquid is water, oil, and vinegar.

24. A bread obtained using a preparation according to any of claims 1 to 18 or a dough according to any of claims 19 to 23.

25. A prebaked bread obtained using a preparation according to any of claims 1 to 18 or a dough according to any of claims 19 to 23.

26. Use of gluten in combination with alpha-cyclodextrin for the preparation of a bread with reduced glycemic index.
